# EUROPEAN PATENT APPLICATION

(11) **EP 3 117 826 A1**
(43) Date of publication of application: **18.01.2017**
(21) Application number: 14885289.0
(22) Date of filing: 11.03.2014
(51) Int. Cl.: A61K 31/465, A61K 9/20

(54) **TOBACCO SUBSTITUTE PRODUCT**

(71) Applicant: Farmalider, S.A., 28108 Alcobendas (Madrid) (ES); Laboratorios Viñas, S.A., 08025 Barcelona (ES)
(72) Inventor: SANZ MENEDEZ, Nuria, 28108 Alcobendas (Madrid) (ES); RIZO MARTINEZ, José Miguel, 28108 Alcobendas (Madrid) (ES); BUXADE VIÑAS, Antonio, 08025 Barcelona (ES); GOMEZ CALVO, Antonia, 28108 Alcobendas (Madrid) (ES); JULIAN PRAT, Ramón, 28108 Alcobendas (Madrid) (ES)
(74) Representative: Rodriguez Oca, Jesus
(86) International application number: PCT/ES2014/000033
(87) International publication number: WO 2015/136124

(57) **Abstract**

The invention relates to a soft nicotine pastille containing nicotine or a nicotine derivative, gelatine, a flavouring agent and an anti-oxidizing agent, and to a tobacco substitute product comprising the said soft nicotine pastille, wherein a stick, that may be used as a handle, has been partly inserted.

## Description

### Technical field

The present invention relates to a tobacco substitute product consisting of a soft nicotine pill with a soft consistency and which comprises a stick.

### Prior art

The damaging effects of smoking are very well known and, among the diseases more frequently related to smoking, lung cancer, chronic obstructive pulmonary disease (COPD), ischemic heart disease and cerebrovascular disease, among others, can be mentioned. However, smoking cessation usually results very difficult, as a consequence of the addictive effects of nicotine.

Nicotine is a natural alkaloid which is obtained from the leaves of tobacco plants. Cigarettes are an extremely efficient system for the administration of nicotine, so that this latter substance is quickly absorbed, arriving to the brain, where receptors are located, only some 10 seconds after the inhalation of smoke, and its effect, at brain level, is one of the contributing factors to the high addictiveness of nicotine.

Pharmacological treatments are frequently used to help to combat anxiety and the withdrawal symptoms experienced during the smoking cessation process, since they have proven to be effective and increase the chances to move away from tobacco dependence in the long term, as it has been described, for instance, in the article written by Wu et al., Effectiveness of smoking cessation therapies: a systematic review and meta-analysis, BMC Public Health; 2006, 6, 300.

Among these pharmacological treatments, we could mention the nicotine-based replacement therapy, consisting of the administration of pure nicotine through any route other than cigarette consumption, in sufficient quantities to relieve withdrawal symptoms, but not to create addiction.

There are different products available in the market that can be used as tobacco replacement products, sold as patches, gum, nasal sprays, inhalers or pills.

All of them are based on the same principle of providing stable levels of nicotine to the patient and, consequently, the selection of a particular choice will mainly depend on the specific characteristics of each individual. For instance, patches may be contraindicated for individuals with dermatological conditions, and gum may not be suitable for those who suffer from jaw alterations or dental disorders. Therefore, it would be desirable to have a sufficient number of alternatives that may be tailored to the needs of each patient in the most appropriate manner.

Certain tobacco replacement products, in the form of a soft pastille with a soft consistency, have been described in the art. Thus, for instance, the British patent application GB-A-2299756 describes gelatinous pastilles containing nicotine in an acacia gum or gelatine base, and optionally, other components such as vitamins, sweeteners and flavourings.

The international patent application WO-A-91/06288 describes pastilles made of gelatine, sugar and glucose that contain nicotine and form a stimulant unit, to be given orally, which releases nicotine in a controlled manner.

Similarly, the international patent application WO-A-2011/101860 describes soft pastilles containing from 0.05 to 1 % of nicotine, from 5 to 40% of a gelling agent, from 30 to 70% of a plasticizer substance, from 0.05 to 10% of a sweetener, from 0.5 to 30% of a releasing agent, from 0.05 to 2% of a preservative, from 0.01 to 5% of a flavouring agent and from 5 to 20% of water, so that such pills are dissolved in the oral cavity within an interval ranging from 5 to 15 minutes.

On the other hand, it is well known that tobacco addiction not only entails a physical or pharmacological dependence caused by nicotine, but also a psychological and behavioural dependence. It is for this reason that those individuals who have quit smoking frequently need to distract their hands in any way to replace the presence of a cigarette.

Thus, some nicotine-replacing therapies have been described in an effort to tackle with this twofold dependence. Thus, for instance, US patent US5048544 describes a candy comprising a stick to use them as a lollypop. The edible part contains a small amount of nicotine and consists of a hard or semi-hard candy, while the stick has the size, shape and consistency of a cigarette.

Consequently, there is a need to have an alternative tobacco substitute which efficiently provides an appropriate amount of nicotine for combating nicotine addiction in those individuals who are quitting smoking and which also may alleviate the psychological and behavioural dependency of former smokers.

### Object of the invention

The object of the present invention relates to a soft nicotine pastille.

Another object of the invention is to provide a tobacco substitute product consisting of the said soft pastille with a stick inserted on it.

### Detailed description of the invention

The object of the present invention is a soft nicotine pastille, containing:
a) Nicotine or a nicotine derivative in a quantity ranging between 0.05% and 1%;
b) Gelatine, in an amount ranging between 20% and 50%;
c) A flavouring agent, in an amount ranging between 20% and 50%; and
d) An anti-oxidizing agent, in an amount ranging between 0.025% and 0.2%;
wherein percentages are expressed in weight with respect to the total weight of the soft pastille.

The authors of the present invention have developed a tobacco substitute product consisting of a soft pastille with a stick which is used as a handle, so that such handle allows users to hold the product in their hands for an intermittent consumption, which contributes to alleviate the psychological and behavioural dependency, while the composition and consistency of the soft nicotine pastille are especially satisfactory from an organoleptic point of view.

### Nicotine

Nicotine is the common designation which is normally used for the product (S)-3-[1-Methylpyrrolidin-2-yl]pyridine. It is an alkaloid that can be found in the nightshade family of plants (*Solanaceae*), especially in the tobacco plant (*Nicotiana tabacum*), as well as in the *Nicotiana rustica.*

Nicotine can be extracted from the tobacco plant, for instance, as described in the book by L. Gattermann y H. Wieland, Laboratory Methods of Organic Chemistry, 24th Edition, New York, 1937, page 406; but it can also be synthesized, as described in the article written by L.C. Craig, A new synthesis of nornicotine and nicotine, 1933, 55(7), 2854-2857; however, it can also be commercially obtained from different vendors like Sigma-Aldrich, for instance.

As used in the present invention, Nicotine can be presented as a free base or as a nicotine derivative. The most appropriate nicotine derivatives, within the scope of this invention, include pharmaceutically acceptable nicotine salts, such as Monotartrate, Bitartrate, Bitartrate Dihydrate, or Sulphate, among others; inclusion complexes with cyclodextrines; solvates; complexes with a resin, wherein the nicotine is bound to an ion exchange resin, for instance, nicotine polacrilex; or even enriched vegetable extracts, among others.

In a preferred embodiment of the invention, the nicotine derivative used is nicotine polacrilex.

Nicotine polacrilex is a cation-exchange resin complex prepared with polymethacrilic acid and divinylbenzene, bound to the nicotine. The preparation of nicotine polacrilex is described, for instance, in the US Patent US3901248 and can be commercially obtained from several suppliers, among them the company Nicobrand.

The composition of the soft nicotine pastilles according to the invention contains nicotine or a nicotine derivative, in percentages ranging between 0.05% and 1% by weight, compared with the total weight of the soft pastille, expressed as the equivalent amount of nicotine, preferably between 0.1 % and 0.5%, and specifically between 0.2% and 0.3%.

In a preferred embodiment, the soft pastille contains an amount of nicotine or a nicotine derivative ranging from 1 mg to 5 mg, expressed as the equivalent weight of nicotine, especially ranging from 1.5 mg to 4.5 mg and specifically from 2 mg to 4 mg.

### Other components

The soft pastilles of the present invention contain gelatine, in a percentage (by weight) ranging from 20% and 50% compared to the total weight of the soft pastille, preferably from 25% to 45%, especially from 30% to 40% and specifically from 33% to 37%.

The composition of the soft pastilles according to the invention also contains a flavouring agent, in a percentage (by weight) ranging from 20% to 50% compared to the total weight of the soft pastille, preferably from 25% to 35%, and specifically from el 27% to 32%.

Among the most suitable flavouring agents that may be used with this invention, sorbitol, maltitol, manitol, erythritol, dextrose, maltose, xylitol and mixtures of them may be mentioned.

In a preferred embodiment of the invention, the flavouring agent used is sorbitol.

Furthermore, the soft pastilles according to the invention contain an anti-oxidizing substance, in a percentage ranging between 0.025% and 0.2% (by weight) of the total weight of the soft pastille, preferably between 0.04% and 0.1 %, and especially between 0.045% and 0.055%.

Among the anti-oxidizing agents that are most suited to be used within the scope of the present invention, butylhydroxyanisol, alpha-tocopherol, ascorbic acid, methionine and butylhydroxytoluene, for instance, as well as their mixtures, could be mentioned.

In a preferred embodiment of the invention, the anti-oxidizing agent used is butylhydroxyanisol.

### Additional components

Additionally, the nicotine soft pastilles according to the invention may contain a preservative, usually comprised within a percentage ranging from 0.05% and 0.2% (by weight) of the total weight of the soft pastille, preferably between 0.075% and 0.15%, and especially between 0.09% and 0.12%.

Such preservative may be chosen among benzoic acid, parabens, (methyl paraben, ethyl paraben, propyl paraben or butyl paraben) or their sodium and potassium salts, benzalkonium chloride or benzyl alcohol.

Also, the soft nicotine pastilles according to the invention may contain a sweetener, usually in an amount comprised between 0.1 % and 0.5% (by weight) of the total weight of the soft pastille, preferably between 0.2% and 0.4%, and especially between 0.25% and 0.3%.

Among the most suitable sweeteners that may be used in this invention, saccharine, sucrose, sucralose, aspartame, acesulfame potassium, trehalose and their mixtures, for instance, can be mentioned.

Furthermore, such soft nicotine pastilles may contain a pH regulator or buffer agent, usually in an amount comprised between 2.5% and 7.5% (by weight) of the total weight of the soft pastille, preferably between 3% and 6%, and especially between 4% and 5.5%.

Among the different pH regulators that are most suitable to be incorporated into the composition of the soft pastilles, dibasic sodium phosphate, monobasic sodium phosphate, sodium carbonate, sodium bicarbonate, monoethanolamine, diethanolamine and triethanolamine, ammonia solution, calcium carbonate, calcium hydroxide and their mixtures, for instance, can be mentioned.

In a preferred embodiment of the invention, the soft pastille also comprises a preservative, a sweetener and a pH regulator.

Thus, a preferred embodiment of the invention is a soft pastille containing:
a) nicotine polacrilex in an amount comprised between 0.05% and 1%, expressed as nicotine equivalent;
b) gelatine in an amount comprised between 20% and 50%;
c) sorbitol in an amount comprised between 20% and 50%;
d) an anti-oxidizing agent, in an amount comprised between 0.025% and 0.2%;
e) a preservative, in an amount comprised between 0.05% and 0.2%;
f) a sweetener, in an amount comprised between 0.1 % and 0.5%; and
g) a pH regulator, in an amount comprised between 2.5% and 7.5%;
where percentages are expressed in weight, compared to the total weight of the soft pastille, and the combined percentages equal to 100%.

Additionally, the soft nicotine pastille according to the invention may also contain an aromatic agent, either natural, like essential oils or natural flavours, or an artificial aromatic additive to provide distinct flavours, for instance mint flavour, eucalyptus, menthol, lemon, orange, anise, strawberry or pineapple, among others.

### Tobacco substitute product

The object of the present invention comprises a tobacco substitute product consisting of a soft pastille according to the invention, which has a stick that has been partly inserted on it, to be used as a support or handle.

It is to be understood as a product aimed at the pharmacological treatment of the dependence on nicotine in those individuals who are quitting smoking, and which is based on the administration of pure nicotine through a route other than cigarette consumption, in sufficient quantities to relieve withdrawal symptoms, but not to create addiction.

The tobacco substitute product according to the present invention resembles those candies or sweets of the "lollypop" or "candy cane" type, which have a stick partly inserted on them, of an appropriate length to be used as a handle, allowing users to keep the candy in their hands, introducing them in their mouth from time to time, for an intermittent consumption.

The stick used with the product according to the invention is usually made of plastic or cardboard, with a rigid or semi-rigid consistency.

In a preferred embodiment of the invention, the tobacco substitute product consists of a soft pastille which has a stick partly inserted on it, that may be used as a handle, and characterised in that the said soft pastille comprises:
a) nicotine or nicotine polacrilex in an amount comprised between 0.05% and 1%;
b) gelatine in an amount comprised between 20% and 50%;
c) sorbitol in an amount comprised between 20% and 50%;
d) an anti-oxidizing agent, in an amount comprised between 0.025% and 0.2%;
e) a preservative, in an amount comprised between 0.05% and 0.2%;
f) a sweetener, in an amount comprised between 0.1 % and 0.5%; and
g) a pH regulator, in an amount comprised between 2.5% and 7.5%, where percentages have been expressed in weight with respect to the total weight of the soft pastille.

In a especially preferred embodiment of the invention, the tobacco substitute product of the present invention also comprises a flavouring agent.

It has been observed that the use of the tobacco substitute product according to the present invention allows the administration of nicotine through a route other than cigarette consumption, in sufficient quantities to relieve withdrawal symptoms, while also alleviating the psychological and behavioural dependency, as it allows users to hold the product in their hands for an intermittent consumption.

### Preparation of the compositions according to the invention

An appropriate procedure for the preparation of the tobacco substitute product that is the object of this invention comprises the following stages:
a) Dissolving gelatine in purified water, previously heated at a temperature ranging between 50°C and 90°C;
b) Adding the anti-oxidizing agent to the previous mixture;
c) Adding the nicotine or the nicotine derivative and the flavouring agent; and
d) Pouring the mixture in moulds, adding the stick and leaving it to cool to room temperature.

Optionally in any of the stages a), b) or c) an additional component, chosen among the group consisting of sweeteners, preservatives, pH regulators, aromatic additives and their mixtures may be incorporated.

The following paragraphs contain, for illustration purposes and without limitation, some exemplary embodiments of the invention.

### Examples

### Example 1: Tobacco substitute product

A tobacco substitute product according to the present invention was prepared, using the following ingredients:

| Ingredient | Amount (g) | % by weight |
|---|---|---|
| Nicotine polacrilex (nicotine equivalent) | 5.9 (1.18) | 0.59 (0.12) |
| Pharma gelatin 100 PS 30 | 320 | 32 |
| Liquid maltitol | 315 | 31.5 |
| Ascorbic acid | 0.5 | 0.05 |
| Propyl paraben | 1.0 | 0.1 |
| Sodium Saccharin | 2.6 | 0.26 |
| Spearmint | 6.0 | 0.6 |
| Purified water | 299 | 29.9 |
| Monoethanolamine | 50.0 | 5 |
| TOTAL | 1000 | 100 |

Purified water was heated at 75°C and propyl paraben and saccharin were firstly added, and then gelatin, stirring until an homogeneous mixture was obtained, Then, ascorbic acid and monoethanolamine were added under stirring, and finally, nicotine, maltitol and mint were added to the mixture, stirring it until complete homogenisation was achieved.

The mixture was poured in a mould in a proportion of 1.7 g per unit, which approximately equals 2 mg of nicotine per each soft pastille, and then a stick to be used as a handle was inserted, and the mixture was allowed to cool.

The soft pastilles with the stick inserted were released from the moulds and allowed to dry at room temperature.

### Example 2: Tobacco substitute product

A tobacco substitute product according to the present invention was prepared, using the following ingredients:

| **Ingredient** | **Amount (g)** | **% by weight** |
|---|---|---|
| Nicotine polacrilex (nicotine equivalent) | 5.9 (1.18) | 0.59 (0.12) |
| Pharma gelatin 100 PS 30 | 380 | 38 |
| Liquid sorbitol | 260 | 26 |
| Ascorbic acid | 0.5 | 0.05 |
| Nipagin M sodium | 1.0 | 0.1 |
| Aspartame | 2.5 | 0.25 |
| Eucalyptus oil | 6.1 | 0.61 |
| Purified water | 294 | 29.4 |
| Dibasic sodium phosphate | 50.0 | 5 |
| Monobasic sodium phosphate | 0.05 | 0.005 |
| TOTAL | 1000 | 100 |

This composition was prepared following a procedure similar to the one used in the Example 1 above, but using sorbitol as flavouring agent, Nipagin M sodium as preservative, aspartame as sweetener, eucalyptus oil as aromatic substance and a mixture of dibasic sodium phosphate and monobasic sodium phosphate as pH regulator, instead of maltitol, propyl paraben, saccharine, spearmint and monoethanolamine, respectively.

### Example 3: Tobacco substitute product

A tobacco substitute product according to the present invention was prepared, using the following ingredients:

| **Ingredient** | **Amount (g)** | **% by weight** |
|---|---|---|
| Nicotine polacrilex (nicotine equivalent) | 5.9 (1.18) | 0.59 (0.12) |
| Gelatin pharma 100 PS 30 | 315 | 31.5 |
| Liquid Sorbitol | 310 | 31.0 |
| Butylhydroxyanisol | 0.5 | 0.05 |
| Nipagin M sodium | 1.0 | 0.1 |
| Sodium saccharine | 3.0 | 0.3 |
| Spearmint | 7.6 | 0.76 |
| Purified water | 307 | 30.7 |
| Dibasic sodium phosphate | 50.0 | 5 |
| Monobasic sodium phosphate | 0.05 | 0.005 |
| TOTAL | 1000.05 | 100 |

This composition was also prepared following a procedure similar to the one used in the Example 1 above, but using sorbitol as flavouring agent, butylhydroxyanisol as anti-oxidizing agent, Nipagin M sodium as preservative and a mixture of dibasic sodium phosphate and monobasic sodium phosphate as pH regulator, instead of maltitol, ascorbic acid, propyl paraben and monoethanolamine, respectively.

## Claims

1. A soft nicotine pastille, **characterised in that** it comprises:
a) nicotine or a nicotine derivative, in an amount comprised between 0.05% and 1 %;
b) gelatin, in an amount comprised between 20% and 50%;
c) a flavouring agent, in an amount comprised between 20% and 50%; and
d) an anti-oxidizing agent, in an amount comprised between 0.025% and 0.2%; wherein percentages have been expressed by weight with respect to the total weight of the a soft pastille.

2. A soft pastille according to claim 1, **characterised in that** the nicotine derivative is nicotine polacrilex.

3. A soft pastille according to claim 1 or 2, **characterised in that** it contains an amount of nicotine comprised between 1 mg and 5 mg, expressed as the equivalent weight of nicotine.

4. A soft pastille according to any of the claims 1 to three, **characterised in that** the flavouring agent is selected among the group comprising sorbitol, maltitol, manitol, erythritol, dextrose, maltose, xylitol and their mixtures.

5. A soft pastille according to claim 4, **characterised in that** the flavouring agent is sorbitol.

6. A soft pastille according to any of the claims 1 to 5, **characterised in that** the anti-oxidizing agent is selected among the group comprising butylhydroxyanisol, alpha-tocopherol, ascorbic acid, methionine, butylhydroxytoluene and their mixtures.

7. A soft pastille according to claim 6, **characterised in that** the flavouring agent used is butylhydroxyanisol.

8. A soft pastille according to any of the claims 1 to 7, **characterised in that** it additionally comprises a preservative.

9. A soft pastille according to any of the claims 1 to 8, **characterised in that** it additionally comprises a sweetener.

10. A soft pastille according to any of the claims 1 to 9, **characterised in that** it additionally comprises a pH regulator.

11. A soft pastille according to claim 10, **characterised in that** the pH regulator is selected among the group comprising dibasic sodium phosphate, monobasic sodium phosphate, sodium carbonate, sodium bicarbonate, monoethanolamine, diethanolamine and triethanolamine, ammonia solution, calcium carbonate, calcium hydroxide and their mixtures.

12. A soft pastille according to any of the claims 1 to 11, **characterised in that** it comprises:
a) nicotine polacrilex in an amount comprised between 0.05% and 1%, expressed as nicotine equivalent;
b) gelatin in an amount comprised between 20% and 50%;
c) sorbitol in an amount comprised between 20% and 50%;
d) an anti-oxidizing agent, in an amount comprised between 0.025% and 0.2%;
e) a preservative, in an amount comprised between 0.05% and 0.2%;
f) a sweetener, in an amount comprised between 0.1 % and 0.5%; and
g) a pH regulator, in an amount comprised between 2.5% and 7.5%;
wherein percentages are expressed in weight, compared to the total weight of the soft pastille.

13. A soft pastille according to any of the claims 1 to 12, **characterised in that** it additionally contains an aromatic agent.

14. Tobacco substitute product **characterised in that** it consists of a soft pastille according to any of the claims 1 to 13, wherein a stick which may be used as a handle has been partly inserted.

15. A procedure to prepare the tobacco substitute product according to claim 14, **characterised in that** it comprises the following stages:
a) Dissolving gelatine in purified water, previously heated at a temperature ranging between 50°C and 90°C;
b) Adding the anti-oxidizing agent to the previous mixture;
c) Adding the nicotine or the nicotine derivative and the flavouring agent; and
d) Pouring the mixture in moulds, inserting the stick and leaving it to cool to room temperature.

16. A procedure according to claim 15, **characterised in that** an additional ingredient selected among the group comprising sweeteners, preservatives, pH regulators, aromatic additives and their mixtures is added in any of the stages a), b) or c).
